# EUROPEAN PATENT SPECIFICATION

(11) **EP 2 007 746 B1**
(45) Date of publication and mention of the grant of the patent: **31.08.2016**
(21) Application number: 07727828.1
(22) Date of filing: 05.04.2007
(51) Int. Cl.: C07D 309/10, C07H 7/00, A61P 17/06, A61P 17/00, A61K 31/351, A61K 8/60

(54) **USE OF A GALACTOSE-DERIVED C-GLYCOSIDE COMPOUND AS AN AGENT FOR ACTIVATING AND REGULATING CUTANEOUS IMMUNITY**
VERWENDUNG EINER VON GALACTOSE ABGELEITETEN C-GLYCOSID-VERBINDUNG ALS MITTEL ZUR AKTIVIERUNG UND STEUERUNG DER CUTANEN IMMUNITÄT
UTILISATION D'UN COMPOSÉ C-GLYCOSIDE DÉRIVÉ DU GALACTOSE EN TANT QU'AGENT POUR ACTIVER ET RÉGULER L'IMMUNITÉ CUTANÉE

(30) Priority: 07.04.2006 FR 0651273; 04.05.2006 US 797381 P
(43) Date of publication of application: 31.12.2008
(73) Proprietor: L'Oréal, 75008 Paris (FR)
(72) Inventor: PINEAU, Nathalie, F-95220 Herblay (FR); DALKO, Maria, F-91190 GIF S/Yvette (FR)
(74) Representative: Touroude, Magali Linda
(86) International application number: PCT/EP2007/053360
(87) International publication number: WO 2007/116013

(56) References cited:
- EP-A- 1 345 919
- WO-A-02/051803
- WO-A-2004/094445
- US-A1- 2002 107 224
- TAKAHASHI S ET AL: "Total Synthesis of an Antitumor Agent , Mucocin, Based on the Chiron Approach" JOURNAL OF ORGANIC CHEMISTRY, AMERICAN CHEMICAL SOCIETY. EASTON, US, vol. 67, no. 16, 7 September 2002 (2002-09-07), pages 5739-5752, XP002262276 ISSN: 0022-3263
- AUGUSTIN ET AL: "C-Glycoside analogues of beta-galactosylceramide with a simple ceramide substitute: Synthesis and binding to HIV-1 gp120" BIOORGANIC & MEDICINAL CHEMISTRY, ELSEVIER SCIENCE LTD, GB, vol. 14, no. 4, 15 February 2006 (2006-02-15), pages 1182-1188, XP005237573 ISSN: 0968-0896
- CHAULAGAIN M R ET AL: "Synthesis and anti-tumor activity of beta-C-glycoside analogs of the immunostimulant KRN7000" TETRAHEDRON LETTERS, ELSEVIER, AMSTERDAM, NL, vol. 45, no. 41, 4 October 2004 (2004-10-04), pages 7791-7794, XP004570501 ISSN: 0040-4039
- TIWARI ET AL: "Acylation of carbohydrates over Al2O3: preparation of partially and fully acylated carbohydrate derivatives and acetylated glycosyl chlorides" CARBOHYDRATE RESEARCH, ELSEVIER SCIENTIFIC PUBLISHING COMPANY. AMSTERDAM, NL, vol. 341, no. 3, 27 February 2006 (2006-02-27), pages 339-350, XP005250473 ISSN: 0008-6215

## Description

The present invention relates to novel galactose-derived C-glycoside compounds useful as agents for stimulating the immune system of the skin and/or as immunoregulators, and to their use for preparing a composition containing a cosmetically or pharmaceutically acceptable medium, intended in particular to prevent and/or limit the appearance of cutaneous immune imbalances, in particular related to environmental stresses.

Cutaneous immune disorders are normal physiological phenomena which appear with age. They can, however, be accelerated by infections with microorganisms (viruses and bacteria), stress, chronological ageing, ultraviolet rays, "urban" living conditions, etc.

The immune system comprises a collection of specialized cells which are subject to multiple control mechanisms that ensure their renewal, their activation and their differentiation, and are essential to a normal level of immunocompetence. The role of the immune system is to discriminate self from non-self in order to eliminate pathogenic agents and spontaneous tumours. Any cellular depletion, any incorrect immune regulation or any functional deficiency is liable to promote the occurrence of manifestations which range from discomfort to pathological disorders characterized by the disturbance of the mechanisms of recognition of self with respect to non-self, and a greater sensitivity with respect to microbial attacks and neoplastic processes.

The skin is an organ that is highly important for the organism and is recognized as one of the main active elements of the immune defence system. Three epidermal cell types participate in this system: keratinocytes, melanocytes and Langerhans cells. These cells, which are found only in the skin, play an essential role in the immune response, and in particular in antigen presentation.

Normal skin constitutes a barrier and is capable of defending itself against outside attacks, in particular chemical and mechanical attacks; in this respect, a certain number of defence reactions against environmental factors (climate, ultraviolet rays, tobacco, pollutants, etc.) and/or xenobiotics (such as, for example, certain medicaments) occur therein.

Various factors, such as atmospheric pollutants, detergents, allergens, UV radiation, etc., negatively affect, by virtue of their action on the skin, a variety of immune responses, both locally at the site of exposure, and systemically, at distant sites. This form of immunosuppression is in particular related to the induction of antigen-specific suppressor T cells. Impairment of the delayed response is particularly important since immune reactions generated by T lymphocytes are responsible for protection against many chronic infectious pathologies.

Pathologies also exist which are based not on an insufficiency of immune cells, but on an immune imbalance; this is the case, in particular, of atopic diseases and autoimmune diseases which present, respectively, an excess of Th-2 lymphocytes and an excess of Th-1 lymphocytes.

The prevalence of atopic diseases (accompanied by an excessive presence of Th-2-type lymphocytes, such as atopic dermatitis, gastrointestinal allergies, allergic rhinitis and conjunctivitis, asthma) and of autoimmune diseases (accompanied by an excessive presence of Th-1-type lymphocytes, such as psoriasis, vitiligo, diffuse scleroderma, lupus erythematosus, certain forms of alopecia, rheumatoid arthritis, type I diabetes) has gradually increased over the last few decades in western societies.

The explanation which has appeared to be the most plausible regarding the increase in Th-2-related conditions is the hygiene-related hypothesis which suggests that the rapid increase in atopic eczemas is related to the current cleanliness of environments and to the decrease in exposure to microorganisms at the beginning of life (Holt PG, in Nestle Nutrition Workshop series Pediatric Program, Isolauri E et al ed, Allergic diseases and the environment, Karger AG, Basel, vol 53 pp 53-68, 2004).

During the allergic reaction, which can be explained by a reorientation of Th-1-type immune reactions towards Th-2-type responses, the interaction between the normal host and the allergen is altered. The allergic reaction is then accompanied by an imbalance in the immune response, which may then be induced by resident bacteria (Martinez FD, Respir Res: 2:129-132, 2001).

These atopic conditions are chronic and often systemic inflammatory reactions of complex origin (genetic and environmental factors). In these pathologies, the responses of type 2 (Th-2) T helper cells to "inoffensive" antigens (allergens) of the environment play a determining role in triggering allergic conditions (Romagnani S, Curr Opin Immunol 6:838-846, 1994). Th-2 cells explain the joint intervention, in the allergic inflammatory process, of B cells that produce E immunoglobulins (via the production of interleukins IL-4 and IL-13), and of mast cells (via the production of IL-5).

Moreover, it is also important to emphasize that, while there is currently an increase in allergic pathologies related to Th-2-type cells, at the same time, an increase in pathologies related to Th-1 cells (autoimmune diseases, such as type I diabetes, psoriasis or vitiligo) is observed in developing countries.

Th-1-type cells play an important role in the development of the delayed hypersensitivity reaction (DHR); thus, in certain chronic autoimmune diseases such as rheumatoid arthritis and thyroiditis, the skin lesions observed are of the DHR type, and the CD4 T cells within said lesions are mainly of the Th-1 type. Identical results have been obtained over the course of infectious diseases due to mycobacteria (tuberculosis, leprosy), over the course of Lyme disease and over the course of psoriasis.

Vitiligo is an acquired depigmentation disorder of the skin that affects 1% of the world's population, regardless of skin colour. Vitiligo is a skin disease in which the melanocytes (MCs) are eliminated from the basal layer of the epidermis in the lesions. This disappearance of melanocytes leads to a deficient pigmentation. In vitiligo lesions, the melanocytes are destroyed by MC-reactive T cells. The depigmentation frequently begins during adolescence.

For example, after an infection, UV radiation or a chemical/mechanical attack, the melanocytes are damaged. Under conditions of normal immune control, these impairments are controlled by the immune system which destroys the modified cells. In the case of vitiligo, these impairments are not correctly treated and they constitute a source of autoantibodies which will contribute to establishing the autoimmune pathology.

Thus, it appears that a therapy which would make it possible to reorient the Th-2 "allergic" or Th-1 "autoimmune" immune response towards a physiological balance would lead to products whose topical application could induce a regulation of local immune phenomena.

The applicant has now demonstrated that C-glycoside compounds of general formula (I) are capable both of stimulating the immune system of the skin and also of rectifying an immune imbalance between populations of Th-1 and Th-2 lymphocytes, and are capable of causing atopic or autoimmune disorders.

It is known that certain sugars such as aldoses, ketoses, deoxyoses or monosaccharide derivatives stimulate immune defences (EP 0 818 201).

O-glycoside or C-glycoside molecules which modulate the immune system also exist, such as C-glycolipid compounds (WO 2003/105769), fucopeptides and amido-deoxygalactose derivatives (US 5,962,660 and WO 96/29339).

The present specification describes the use of compounds of general formula (I'): 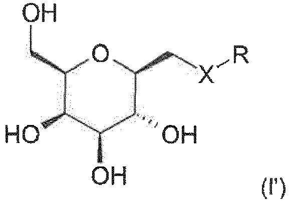 in which,
- X represents a group chosen from: -CO-, -CH(NR₁R₂)-, -CHR'- and -C(=CHR')-;
- R represents a linear or branched, saturated or unsaturated alkyl, perfluoroalkyl or hydrofluoroalkyl chain, or a cycloalkyl, cycloperfluoroalkyl or cyclohydrofluoroalkyl ring, containing from 1 to 18 carbon atoms, or a phenyl radical, it being possible for said chain, said ring or said radical to be optionally interrupted with one or more heteroatoms chosen from oxygen, sulphur, nitrogen and silicon, and optionally substituted with at least one radical chosen from -OR'₁-; -SR"₁, -NR"'₁R'₂, -COOR"₂, -CONHR'"₂, -CN, halogen, perfluoroalkyl and hydrofluoroalkyl, and/or at least one cycloalkyl, aryl or heterocyclic radical, optionally substituted;
- R', R₁ and R₂, which may be identical or different, have the same definition as that given for R, and can also represent a hydrogen and a hydroxyl radical;
- R'₂ and R"'₂, which may be identical or different, represent a hydrogen atom, or a radical chosen from a hydroxyl radical and a linear or branched, saturated or unsaturated alkyl, perfluoroalkyl and/or hydrofluoroalkyl radical containing from 1 to 20 carbon atoms;
- R'₁, R"₁, R"₂ and R"'₁, which may be identical or different, represent a hydrogen atom, or a radical chosen from a linear or branched, saturated or unsaturated alkyl, perfluoroalkyl and/or hydrofluoroalkyl radical containing from 1 to 20 carbon atoms;
with the following restrictions:
- R1 and R2 cannot simultaneously be a hydroxyl radical;
- R'2 and R"'1 cannot simultaneously be a hydroxyl;
for combating the weakening of the natural defences of the skin which appears during chronological or photoinduced ageing and/or reinforcing the natural defences of the skin.

Preference will be given to the compounds of general formula (I') as defined above, such that:
- R represents a linear or branched, saturated or unsaturated alkyl chain, or a cycloalkyl ring containing from 1 to 10 carbon atoms, or a phenyl radical, it being possible for said chain, said ring or said radical to be optionally substituted with at least radical chosen from -OR'₁-, -NR'"₁R'₂, -COOR"₂ and CONHR"'₂;
- R'₂ and R"'₂, which may be identical or different, represent a hydrogen atom, or a radical chosen from a hydroxyl radical and a linear or branched, saturated or unsaturated alkyl radical containing from 1 to 8 carbon atoms;
- R'₁, R"₁, R"₂ and R'"₁, which may be identical or different, represent a hydrogen atom, or a linear or branched, saturated or unsaturated alkyl radical containing from 1 to 8 carbon atoms.

More particularly, further preference will be given to the compounds of general formula (I'), such that:
- X represents a group chosen from: -CO-, -CH(NR₁R₂)-and -CHR';
- R represents a linear or branched, saturated or unsaturated alkyl chain, or a cycloalkyl ring, containing from 1 to 10 carbon atoms, or a phenyl radical.

The C-glycoside compounds that can be used according to the invention represent a subfamily of the C-glycoside derivatives described in EP 1 345 919; they can be prepared according to the process described in said document.

Among the C-glycoside derivatives of formula (I') used according to the invention, the following are most particularly preferred:
Compound 2. Phenyl-2-(C-β-D-galactopyranosyl)-1-hydroxyethane; 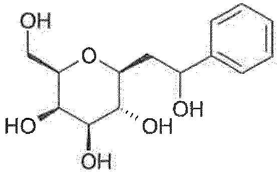
Compound 4. 1-[2-(3-hydroxypropylamino)propyl]-C-β-D-galactopyranose; 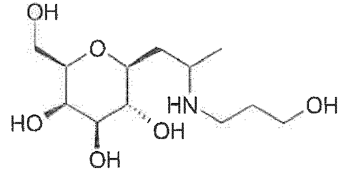
Compound 5. 3-methyl-4-(C-β-D-galactopyranosyl)-2-butenoic acid ethyl ester; 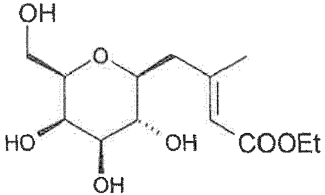
Compound 6. Ethyl (2E)-3-methyl-4-[(2S,3R,4R,5R,6R)-3,4,5-trihydroxy-6-(hydroxymethyl)tetrahydro-2H-pyran-2-yl]but-2-enoate.

More particularly, the use of the C-glycosides of general formulae (I') according to the invention is suitable for preparing the skin against exposure to the sun.

Thus, the use according to the invention makes it possible to prevent and/or limit the harmful effects of exposure to UV rays.

The C-glycosides of general formulae (I') can also be used for maintaining a balance between Th-1 and Th-2 lymphocyte populations and/or for correcting an immune imbalance related to an excess of Th-1-type lymphocytes or Th-2-type lymphocytes.

These compounds according to the invention may therefore be advantageously used for combating undesirable manifestations of atopic type, in particular for treating reactive skin (characterized by red blotches, painful sensations, swelling), for preventing and/or decreasing itching, or else combating autoimmune conditions such as an imbalance in pigmentation of the skin and/or of the hair, in particular hair turning white or grey prematurely.

According to another of its subjects, the present invention relates to the use of C-glycoside compounds of general formulae (I'), for preparing a composition, comprising a physiologically acceptable medium, for use in the prevention and/or treatment of cutaneous autoimmune diseases or cutaneous atopic disorders.

More particularly, the cutaneous atopic disorders are chosen from cutaneous allergic reactions, atopic dermatitis and atopic eczema, and the cutaneous autoimmune diseases are chosen from delayed contact hypersensitivity, psoriasis, vitiligo, diffuse scleroderma, lupus erythematosus or certain forms of alopecia.

The term "immunostimulant agent" is intended to mean a compound whose administration to an organism results in the proliferation of the immune cells of said organism, for example the lymphocytes.

The term "immunoregulatory agent" is intended to mean an agent capable of maintaining and/or reestablishing a cutaneous immune balance between Th-1-type and Th-2-type cell populations, or else of correcting an excessive presence of Th-1-type or Th-2-type cells.

An immune imbalance may in particular be demonstrated by virtue of the increase, in an organism, of one or more cytokines characteristic of a lymphocyte type.

In fact, in addition to their classification according to the structure of their T receptor, Th-1-type and Th-2-type lymphocytes have been classified according to their cytokine protocol.

The cytokines characteristic of type 1 (Th-1) lymphocytes are IL-2, IFN-γ and TNF-β. The cytokines of type 2 (Th-2) lymphocytes are IL-4, IL-5, IL-9, IL-10 and IL-13.

More generally, the C-glycoside compounds of general formulae (I') can be used as an immunostimulant medicament in humans or in animals.

For this type of use, the compositions comprising the C-glycoside compounds of general formulae (I') can be administered, for example, parenterally, (intraperitoneally, subcutaneously, intramuscularly, intravenously, percutaneously), orally, nasally, conjuctivally, rectally or perlingually.

They can also be used by local application, for example by means of orally disintegrating tablets, in particular in non-specific immunotherapy of oral cavity diseases.

The medicament of the invention can be administered by way of prophylaxis, in the various cases above, and in particular for the prevention of recurring infections of the ear, nose and throat (ENT) sphere, and for the prevention of risks of infection in chronically ill patients.

The medicament of the invention is administered in particular as an immunostimulant treatment, in the ENT or bronchopulmonary field (rhinopharyngitis, laryngitis, sinusitis, sore throats, otitis, bronchitis, etc.) or in the dermatological field, in the case of bacterial, fungal or viral infections.

Preferably, the C-glycoside compound of general formulae (I') according to the invention will be formulated in a cosmetic or pharmaceutical composition intended to be applied topically to the skin, the scalp or the mucous membranes.

The compositions used according to the invention can be in any of the forms suitable for the applications envisaged, in particular topical application, in the cosmetics and dermatological fields.

The composition according to the invention contains a physiologically acceptable medium and one or more compounds according to the invention in an effective amount for stimulating the immunity of the skin or for reequilibrating the balance between Th-1 and Th-2 lymphocytes, for example in an amount ranging from 0.01% to 30% by weight, and preferably from 0.1% to 5% by weight, relative to the total weight of the composition.

The term "physiologically acceptable medium" is understood to mean a medium compatible with the skin and, optionally, with the mucous membranes, the nails, the scalp and/or the hair.

The composition according to the invention can be in the form in particular of an aqueous solution or a dispersion of the lotion or serum type, emulsions with a liquid or semi-liquid consistency, of the milk type, obtained by dispersion of a fatty phase in an aqueous phase (O/W) or vice versa (W/O), or suspensions or emulsions which are soft in consistency, of the aqueous or anhydrous gel or cream type, or else microcapsules or microparticles, or vesicular dispersions of ionic and/or non-ionic type. These compositions are prepared according to the usual methods.

This composition may be more or less fluid and may have the appearance of a white or coloured cream, an ointment, a milk, a lotion, a serum, a paste or a foam. It can optionally be applied to the skin in the form of an aerosol. It can also be in the form of a solid, for example in the form of a stick. It can be used as a care product, as a cleansing product, as a makeup product or alternatively as a shampoo or conditioner.

When the composition that can be used according to the invention is an emulsion, the proportion of the fatty phase can range from 5% to 80% by weight, and preferably from 5% to 50% by weight, relative to the total weight of the composition. The oils, the waxes, the emulsifiers and the coemulsifiers used in the composition in the form of an emulsion are chosen from those conventionally used in the cosmetics field. The emulsifier and the coemulsifier are present, in the composition, in a proportion ranging from 0.3% to 30% by weight, and preferably from 0.5% to 20% by weight, relative to the total weight of the composition. The emulsion can also contain lipid vesicles.

The composition according to the invention can be intended for a cosmetic or pharmaceutical, particularly dermatological, application. The composition according to the invention is preferably intended for a cosmetic application.

A subject of the invention is therefore also a cosmetic treatment process for the skin or for the scalp, comprising the topical application to the skin or the scalp of the composition described above.

Given the immunostimulant and equilibrating properties of the compounds according to the invention, this process is, in particular, intended to reinforce the natural defences of the skin and to improve the cutaneous immune balance.

The C-glycoside compounds according to the invention will advantageously be combined with active agents for the hair, chosen from:
- anti-seborrhoeic agents, such as certain sulphur-containing amino acids, 13-cis-retinoic acid or cyproterone acetate;
- agents for combating squamous states of the scalp (dandruff), such as zinc pyrithione, selenium disulphide, climbazole, undecylenic acid, ketoconazole, piroctone olamine (octopirox) or cyclopiroctone (cyclopirox);
- active agents for stimulating hair regrowth and/or promoting the slowing down of hair loss; mention may more particularly be made, in a non-limiting manner, of:
   * nicotinic acid esters, including in particular tocopheryl nicotinate, benzyl nicotinate and C₁-C₆ alkyl nicotinates, for instance methyl nicotinate or hexyl nicotinate;
   * pyrimidine derivatives, such as 2,4-diamino-6-piperidinopyrimidine 3-oxide or "Minoxidil" described in patents US 4,139,619 and US 4,596,812; Aminexil or 2,4-diaminopyrimidine 3-oxide described in WO 96/09048;
   * agents that are both lipoxygenase inhibitors and cyclooxygenase inducers, or cyclooxygenase-inducing agents that promote hair regrowth, such as those described by the applicant in European patent application EP 0 648 488;
- antibiotics such as macrolides, pyranosides and tetracyclines, and in particular erythromycin;
- cinnarizine, nimodipine and nifedipine;
- hormones, such as estriol or the like, or thyroxine and salts thereof;
- antiandrogenic agents, such as oxendolone, spironolactone, diethylstilbestrol and flutamide;
- cromakalim and nicrorandil.

### Example 1 - Demonstration of the immunostimulant activity of the C-glycoside derivatives of the invention

The immunostimulant activity is tested in the following way: human peripheral blood cells are cultured in the presence of an RPMI-type culture medium supplemented with L-glutamine (2 mM), penicillin/streptomycin (50 µg/50 IU/ml) and foetal calf serum (10%). The C-glycoside derivatives are added at various concentrations (10 to 0.05 mM), as is phyto-haemagglutinin (PHA at 5 *G/ml), a positive control for lymphocyte proliferation. After 3 days of culture, the proliferation is revealed by BrdU labelling.

The results obtained are as follows:

| Active agent | % stimulation relative to the control | | | | | |
|---|---|---|---|---|---|---|
| Concentrations (mM) → | 10 | 5 | 1 | 0.5 | 0.1 | 0.05 |
| **Compound 1:** 1-(C-β-D-Galactopyranosyl)propan-2-one | 271 | 261 | 138 | 130 | 89 | 91 |

The derivative tested exhibits a strong capacity for human lymphocyte proliferation.

Compound 1 has a tendency to stimulate human lymphocyte proliferation at all the concentrations tested, this compound therefore has an immunostimulant activity.

### Example 2 - Formulations

**Face care ge1**

| | |
|---|---|
| Compound 1 | 0.05% |
| Thickening polymer | 1.00% |
| Antioxidant | 0.05% |
| Isopropanol | 40.00% |
| Preserving agent | 0.30% |
| Water | qs 100% |

**Face lotion for hyperreactive skin**

| | |
|---|---|
| Compound 6 | 0.50 |
| Magnesium gluconate | 3.00 |
| Antioxidant | 0.05 |
| Isopropanol | 40.0 |
| Preserving agent | 0.30 |
| Water | qs 100% |

## Claims

1. Compounds chosen from:
Compound 2. Phenyl-2-(C-β-D-galactopyranosyl)-1-hydroxyethane;
Compound 4. 1-[2-(3-hydroxypropylamino)propyl]-C-β-D-galactopyranose;
Compound 5. 3-methyl-4-(C-β-D-galactopyranosyl)-2-butenoic acid ethyl ester;
Compound 6. Ethyl (2E)-3-methyl-4-[(2S, 3R, 4R, 5R, 6R)-3,4,5-trihydroxy-6-(hydroxymethyl)tetrahydro-2H-pyran-2-yl]but-2-enoate.

2. Cosmetic or dermatological composition intended to be applied topically, comprising at least one compound according to Claim 1.

3. Composition according to Claim 2, also comprising at least one agent for the hair.

4. Cosmetic or dermatological composition comprising at least one compound of general formula (I'): 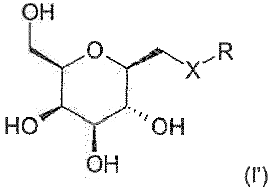 in which:
- X represents a group chosen from: -CO-, -CH(NR₁R₂)-, -CHR'- and -C(=CHR')-;
- R', R₁ and R₂, which may be identical or different, have the same definition as that given for R, and can also represent a hydrogen and a hydroxyl radical;
- R represents a linear or branched, saturated or unsaturated alkyl chain, or a cycloalkyl ring, containing from 1 to 10 carbon atoms, or a phenyl radical, it being possible for said chain, said ring or said radical to be optionally substituted with at least one radical chosen from -OR'₁, -NR"'₁R'₂, -COOR"₂ and -CONHR"'₂;
- R'₂ and R"'₂, which may be identical or different, represent a hydrogen atom, or a radical chosen from a hydroxyl radical and a linear or branched, saturated or unsaturated alkyl radical containing from 1 to 8 carbon atoms;
- R'₁, R"₁, R"₂ and R"'₁, which may be identical or different, represent a hydrogen atom, or a radical chosen from a linear or branched, saturated or unsaturated alkyl radical containing from 1 to 8 carbon atomswith the following restriction:
- R1 and R2 cannot simultaneously be a hydroxyl radical;
Said compound being combined with active agents for the hair, chosen from :
a) 13-cis-retinoic acid or cyproterone acetate;
b) zinc pyrithione, selenium disulphide, climbazole, undecylenic acid, ketoconazole, piroctone, olamine or cyclopiroctone;
c) active agents for stimulating hair regrowth and/or promoting the slowing down of hair loss chosen from :
i) 2,4-diamino-6-piperidinopyrimidine 3-oxide or 2,4-diaminopyrimidine 3-oxide;
ii) 6-chloro-2,3-dihydroxy-1,4-naphthoquinone, , nitric oxide and nitric oxide-donating compounds, stanozolol, anthocyanosides, bioflavonoids, FGA ;
d) cinnarizine, nimodipine and nifedipine;
e) cromakalim and nicrorandil.

5. Non-therapeutic cosmetic use of at least one compound of general formula (I'): 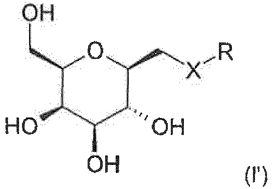 in which:
- X represents a group chosen from: -CO-, -CH(NR₁R₂)-, -CHR'- and -C(=CHR')-;
- R', R₁ and R₂, which may be identical or different, have the same definition as that given for R, and can also represent a hydrogen and a hydroxyl radical;
- R represents a linear or branched, saturated or unsaturated alkyl chain, or a cycloalkyl ring, containing from 1 to 10 carbon atoms, or a phenyl radical, it being possible for said chain, said ring or said radical to be optionally substituted with at least one radical chosen from -OR'₁, -NR"'₁R'₂, -COOR"₂ and -CONHR"'₂;
- R'₂ and R"'₂, which may be identical or different, represent a hydrogen atom, or a radical chosen from a hydroxyl radical and a linear or branched, saturated or unsaturated alkyl radical containing from 1 to 8 carbon atoms;
- R'₁, R"₁, R"₂ and R"'₁, which may be identical or different, represent a hydrogen atom, or a radical chosen from a linear or branched, saturated or unsaturated alkyl radical containing from 1 to 8 carbon atoms
with the following restriction:
- R1 and R2 cannot simultaneously be a hydroxyl radical;
for treating hair turning white or grey prematurely.

6. Use according to Claim 5, **characterized in that**:
- X represents a group chosen from: -CO-, -CH(NR₁R₂)-and -CHR';
- R represents a linear or branched, saturated or unsaturated alkyl chain, or a cycloalkyl ring, containing from 1 to 10 carbon atoms, or a phenyl radical.

7. Use according to any one of Claims 5 to 6, **characterized in that** said compound of general formula (I') is combined with at least one active agent for the hair.

8. Use of at least one compound of general formula (I'): 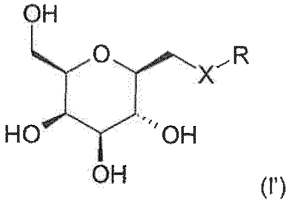 in which:
- X represents a group chosen from: -CO-, -CH(NR₁R₂)-, -CHR'- and -C(=CHR')-;
- R', R₁ and R₂, which may be identical or different, have the same definition as that given for R, and can also represent a hydrogen and a hydroxyl radical;
- R represents a linear or branched, saturated or unsaturated alkyl chain, or a cycloalkyl ring, containing from 1 to 10 carbon atoms, or a phenyl radical, it being possible for said chain, said ring or said radical to be optionally substituted with at least one radical chosen from: -OR'₁-, -NR"'₁R'₂, -COOR"₂ and - CONHR"'₂;
- R'₂ and R'"₂, which may be identical or different, represent a hydrogen atom, or a radical chosen from a hydroxyl radical and a linear or branched, saturated or unsaturated alkyl radical containing from 1 to 8 carbon atoms;
- R'₁, R"₁, R"₂ and R"'₁, which may be identical or different, represent a hydrogen atom, or a radical chosen from a linear or branched, saturated or unsaturated alkyl radical containing from 1 to 8 carbon atoms.
with the following restriction:
- R1 and R2 cannot simultaneously be a hydroxyl radical;
for preparing a composition, comprising a physiologically acceptable medium, for use in the prevention and/or treatment of cutaneous autoimmune diseases or cutaneous atopic disorders.

9. Use according to Claim 8, **characterized in that**:
- X represents a group chosen from: -CO-, -CH(NR₁R₂)-and -CHR'-;
- R represents a linear or branched, saturated or unsaturated alkyl chain, or a cycloalkyl ring, containing from 1 to 10 carbon atoms, or a phenyl radical.

10. Use according to any one of Claims 8 to 9, **characterized in that** the cutaneous atopic disorders are chosen from cutaneous allergic reactions, atopic dermatitis and atopic eczema.

11. Use according to Claim 10, **characterized in that** the cutaneous autoimmune diseases are chosen from delayed contact hypersensitivity, psoriasis, vitiligo, diffuse scleroderma, lupus erythematosus or certain forms of alopecia.

12. Use according to any one of Claims 5 to 11, **characterized in that** said composition is intended to be applied topically to the skin, the mucous membranes or the scalp.

13. Use of at least one compound of general formula (I') chosen from:
Compound 1. 1-(C-β-D-galactopyranosyl)propan-2-one;
Compound 2. Phenyl-2-(C-β-D-galactopyranosyl)-1-hydroxyethane;
Compound 3. 1-(C-β-D-galactopyranosyl)undecan-2-one;
Compound 4. 1-[2-(3-hydroxypropylamino)propyl]-C-β-D-galactopyranose;
Compound 5. 3-methyl-4-(C-β-D-galactopyranosyl)-2-butenoic acid ethyl ester;
Compound 6. Ethyl (2E)-3-methyl-4-[(2S,3R,4R,5R,6R)-3,4,5-trihydroxy-6-(hydroxymethyl)tetrahydro-2H-pyran-2-yl]but-2-enoate,
for treating hair turning white or grey prematurely which appears during chronological or photoinduced ageing.

14. Use of at least one compound of general formula (I') chosen from:
Compound 1. 1-(C-β-D-galactopyranosyl)propan-2-one;
Compound 2. Phenyl-2-(C-β-D-galactopyranosyl)-1-hydroxyethane;
Compound 3. 1-(C-β-D-galactopyranosyl)undecan-2-one;
Compound 4. 1-[2-(3-hydroxypropylamino)propyl]-C-β-D-galactopyranose;
Compound 5. 3-methyl-4-(C-β-D-galactopyranosyl)-2-butenoic acid ethyl ester;
Compound 6. Ethyl (2E)-3-methyl-4-[(2S, 3R, 4R, 5R, 6R)-3,4,5-trihydroxy-6-(hydroxymethyl)tetrahydro-2H-pyran-2-yl]but-2-enoate,
for preparing a composition, comprising a physiologically acceptable medium, for use in the prevention and/or treatment of cutaneous autoimmune diseases or cutaneous atopic disorders.

## Patentansprüche

1. Verbindungen, die aus den folgenden ausgewählt sind:
Verbindung 2. Phenyl-2-(C-β-D-galactopyranosyl)-1-hydroxyethan;
Verbindung 4. 1-(2-(3-Hydroxypropylamino)propyl]-C-β-D-galactopyranose;
Verbindung 5. 3-Methyl-4-(C-β-D-galactopyranosyl)-2-butensäureethylester;
Verbindung 6. Ethyl-(2E)-3-methyl-4-[(2S,3R,4R,5R,6R)-3,4,5-trihydroxy-6-(hydroxymethyl)tetrahydro-2H-pyran-2-yl]but-2-enoat.

2. Kosmetische oder dermatologische Zusammensetzung, die zur topischen Anwendung bestimmt ist, wobei sie mindestens eine Verbindung gemäß Anspruch 1 umfasst.

3. Zusammensetzung gemäß Anspruch 2, welche weiterhin mindestens einen Stoff für das Haar umfasst.

4. Kosmetische oder dermatologische Zusammensetzung, die mindestens eine Verbindung nach der allgemeinen Formel (I') umfasst: 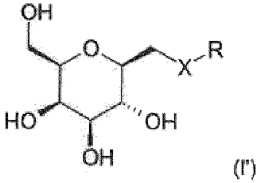 in welcher:
- X für eine Gruppe steht, die aus den folgenden ausgewählt ist: -CO-, -CH(NR₁R₂)-, -CHR'- und - C(=CHR')-;
- R', R₁ und R₂, welche identisch oder verschiedenartig sein können, derselben Begriffsbestimmung entsprechen, wie sie für R angegeben ist, und weiterhin für Wasserstoff oder ein Hydroxylradikal stehen können;
- R für eine geradkettige oder verzweigte, gesättigte oder ungesättigte Alkylkette oder einen Cycloalkylring, welche(r) 1 bis 10 Kohlenstoffatome enthält, oder einen Phenylrest steht, wobei diese Kette, dieser Ring oder dieser Rest möglicherweise mit mindestens einem Rest substituiert sein können, der aus -OR'₁,-NR"'₁R'₂, -COOR"₂ und -CONHR"'₂ ausgewählt ist;
- R'₂ und R"'₂, welche identisch oder verschiedenartig sein können, für ein Wasserstoffatom oder einen Rest stehen, der aus einem Hydroxylrest und einem geradkettigen oder verzweigten, gesättigten oder ungesättigten, Alkylrest ausgewählt ist, welcher 1 bis 8 Kohlenstoffatome enthält;
- R'₁, R"₁, R"₂ und R"'₁, welche identisch oder verschiedenartig sein können, für ein Wasserstoffatom oder einen Rest stehen, der aus einem geradkettigen oder verzweigten, gesättigten oder ungesättigten, Alkylrest ausgewählt ist, welcher 1 bis 8 Kohlenstoffatome enthält, mit der folgenden Einschränkung:
- R1 und R2 können nicht gleichzeitig ein Hydroxylrest sein;
wobei die Verbindung mit Wirkstoffen für das Haar kombiniert ist, die aus den folgenden ausgewählt sind:
a) 13-cis-Retinsäure oder Cyproteronacetat;
b) Zinkpyrithion, Selendisulfid, Climbazol, Undecylensäure, Ketoconazol, Pirocton, Olamin oder Cyclopirocton;
c) Wirkstoffen zur Stimulierung des Nachwachsens von Haar und/oder zur Förderung der Verlangsamung des Haarverlusts, welche aus den folgenden ausgewählt sind:
i) 2,4-Diamino-6-piperidinopyrimidin-3-oxid oder 2,4-Diaminopyrimidin3-oxid;
ii)6-Chlor-2,3-dihydroxy-1,4-naphthochinon, Stickstoffmonoxid und stickstoffmonoxidabgebende Verbindungen, Stanozolol, Anthocyanoside, Bioflavonoide, FGA;
d) Cinnarizin, Nimodipin und Nifedipin;
e) Cromakalim und Nicrorandil.

5. Nicht-therapeutische kosmetische Verwendung mindestens einer Verbindung nach der allgemeinen Formel (I') : 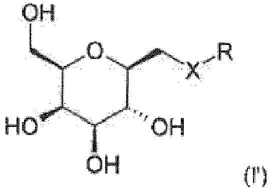 in welcher:
- X für eine Gruppe steht, die aus den folgenden ausgewählt ist: -CO-, -CH(NR₁R₂)-, -CHR'- und - C(=CHR') -;
- R', R₁ und R₂, welche identisch oder verschiedenartig sein können, derselben Begriffsbestimmung entsprechen, wie sie für R angegeben ist, und weiterhin für Wasserstoff oder ein Hydroxylradikal stehen können;
- R für eine geradkettige oder verzweigte, gesättigte oder ungesättigte Alkylkette, oder einen Cycloalkylring, welche(r) 1 bis 10 Kohlenstoffatome enthält, oder einen Phenylrest steht, wobei diese Kette, dieser Ring oder dieser Rest möglicherweise mit mindestens einem Rest substituiert sein können, der aus -OR'₁,-NR"'₁R'₂, -COOR"₂ und -CONHR"'₂ ausgewählt ist;
- R'₂ und R"'₂, welche identisch oder verschiedenartig sein können, für ein Wasserstoffatom oder einen Rest stehen, der aus einem Hydroxylrest und einem geradkettigen oder verzweigten, gesättigten oder ungesättigten Alkylrest ausgewählt ist, welcher 1 bis 8 Kohlenstoffatome enthält;
- R'₁, R"₁, R"₂ und R"'₁, welche identisch oder verschiedenartig sein können, für ein Wasserstoffatom oder einen Rest stehen, der aus einem geradkettigen oder verzweigten, gesättigten oder ungesättigten Alkylrest ausgewählt ist, welcher 1 bis 8 Kohlenstoffatome enthält,
mit der folgenden Einschränkung:
- R1 und R2 können nicht gleichzeitig ein Hydroxylrest sein;
zur Behandlung von Haar, das verfrüht weiß oder grau wird.

6. Verwendung gemäß Anspruch 5, **dadurch gekennzeichnet, dass**:
- X für eine Gruppe steht, die aus den folgenden ausgewählt ist: -CO-, -CH(NR₁R₂) - und -CHR';
- R für eine geradkettige oder verzweigte, gesättigte oder ungesättigte Alkylkette oder einen Cycloalkylring, welche(r) 1 bis 10 Kohlenstoffatome enthält, oder einen Phenylrest steht.

7. Verwendung gemäß einem beliebigen der Ansprüche 5 bis 6, **dadurch gekennzeichnet, dass** die Verbindung nach der allgemeinen Formel (I') mit mindestens einem Wirkstoff für das Haar kombiniert ist.

8. Verwendung mindestens einer Verbindung nach der allgemeinen Formel (I') : 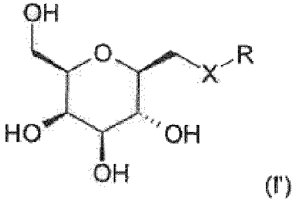 in welcher:
- X für eine Gruppe steht, die aus den folgenden ausgewählt ist: -CO-, -CH(NR₁R₂)-, -CHR'- und - C(=CHR')-;
- R', R₁ und R₂, welche identisch oder verschiedenartig sein können, derselben Begriffsbestimmung entsprechen, wie sie für R angegeben ist, und weiterhin für Wasserstoff oder ein Hydroxylradikal stehen können;
- R für eine geradkettige oder verzweigte, gesättigte oder ungesättigte Alkylkette, oder einen Cycloalkylring, welche(r) 1 bis 10 Kohlenstoffatome enthält, oder einen Phenylrest steht, wobei diese Kette, dieser Ring oder dieser Rest möglicherweise mit mindestens einem Rest substituiert sein können, der aus den folgenden ausgewählt ist: -OR'₁-, -NR"₁R'₂,-COOR"₂ und -CONHR"'₂;
- R'₂ und R"'₂, welche identisch oder verschiedenartig sein können, für ein Wasserstoffatom oder einen Rest stehen, der aus einem Hydroxylrest und einem geradkettigen oder verzweigten, gesättigten oder ungesättigten Alkylrest ausgewählt ist, welcher 1 bis 8 Kohlenstoffatome enthält;
- R'₁, R"₁, R"₂ und R"'₁, welche identisch oder verschiedenartig sein können, für ein Wasserstoffatom oder einen Rest stehen, der aus einem geradkettigen oder verzweigten, gesättigten oder ungesättigten, Alkylrest ausgewählt ist, welcher 1 bis 8 Kohlenstoffatome enthält,
mit der folgenden Einschränkung:
- R1 und R2 können nicht gleichzeitig ein Hydroxylrest sein;
zur Herstellung einer Zusammensetzung, die ein physiologisch unbedenkliches Medium umfasst, zur Verwendung bei der Verhütung und/oder Behandlung von Autoimmunerkrankungen der Haut oder atopischen Störungen der Haut.

9. Verwendung gemäß Anspruch 8, **dadurch gekennzeichnet, dass**:
- X für eine Gruppe steht, die aus den folgenden ausgewählt ist: -CO-, -CH(NR₁R₂)- und -CHR'-;
- R für eine geradkettige oder verzweigte, gesättigte oder ungesättigte, Alkylkette oder einen Cycloalkylring, welche(r) 1 bis 10 Kohlenstoffatome enthält, oder einen Phenylrest steht.

10. Verwendung gemäß einem beliebigen der Ansprüche 8 bis 9, **dadurch gekennzeichnet, dass** die atopischen Störungen der Haut aus allergischen Hautreaktionen, atopischer Dermatitis und atopischem Ekzem ausgewählt sind.

11. Verwendung gemäß Anspruch 10, **dadurch gekennzeichnet, dass** die Autoimmunerkrankungen der Haut aus der verzögerten Kontaktüberempfindlichkeit, Schuppenflechte, Vitiligo, diffuser Sklerodermie, Lupus erythematodes oder bestimmten Formen des Haarausfalls ausgewählt sind.

12. Verwendung gemäß einem beliebigen der Ansprüche 5 bis 11, **dadurch gekennzeichnet, dass** die Zusammensetzung für eine topische Anwendung auf der Haut, den Schleimhäuten oder der Kopfhaut bestimmt ist.

13. Verwendung mindestens einer Verbindung nach der allgemeinen Formel (I'), welche aus den folgenden ausgewählt ist:
Verbindung 1. 1-(C-β-D-Galactopyranosyl)propan-2-on;
Verbindung 2. Phenyl-2-(C-β-D-galactopyranosyl)-1-hydroxyethan;
Verbindung 3. 1-(C-β-D-Galactopyranosyl)undecan-2-on;
Verbindung 4. 1-[2-(3-Hydroxypropylamino)propyl)-C-β-D-galactopyranose;
Verbindung 5. 3-Methyl-4-(C-β-D-galactopyranosyl)-2-butensäureethylester;
Verbindung 6. Ethyl-(2E)-3-methyl-4-[(2S,3R,4R,5R,6R)-3,4,5-trihydroxy-6-(hydroxymethyl)tetrahydro-2H-pyran-2-yl]but-2-enoat,
zur Behandlung der verfrühten Weiß- oder Graufärbung des Haars, wie sie während der zeitbedingten oder lichtinduzierten Alterung auftritt.

14. Verwendung mindestens einer Verbindung nach der allgemeinen Formel (I'), welche aus den folgenden ausgewählt ist:
Verbindung 1. 1-(C-β-D-Galactopyranosyl)propan-2-on;
Verbindung 2. Phenyl-2-(C-β-D-galactopyranosyl)-1-hydroxyethan;
Verbindung 3. 1-(C-β-D-Galactopyranosyl)undecan-2-on;
Verbindung 4. 1-[2-(3-Hydroxypropylamino)propyl]-C-β-D-galactopyranose;
Verbindung 5. 3-Methyl-4-(C-β-D-galactopyranosyl)-2-butensäureethylester;
Verbindung 6. Ethyl-(2E)-3-methyl-4-[(2S,3R,4R,5R,6R)-3,4,5-trihydroxy-6-(hydroxymethyl)tetrahydro-2H-pyran-2-yl]but-2-enoat,
zur Herstellung einer Zusammensetzung, die ein physiologisch unbedenkliches Medium umfasst, zur Verwendung bei der Verhütung und/oder Behandlung von Autoimmunerkrankungen der Haut oder atopischen Störungen der Haut.

## Revendications

1. Composés choisis parmi :
Composé 2. phényl-2-(C-β-D-galactopyranosyl)-1-hydroxyéthane ;
Composé 4. 1-[2-(3-hydroxypropylamino)propyl]-C-β-D-galactopyranose ;
Composé 5. ester éthylique d'acide 3-méthyl-4-(C-β-D-galactopyranosyl)-2-buténoïque ;
Composé 6. (2E)-3-méthyl-4-[(2S,3R,4R,5R,6R)-3,4,5-trihydroxy-6-(hydroxyméthyl)tétrahydro-2H-pyran-2-yl]but-2-énoate d'éthyle.

2. Composition cosmétique ou dermatologique destinée à être appliquée de façon topique, comprenant au moins un composé selon la revendication 1.

3. Composition selon la revendication 2, comprenant en outre au moins un agent pour les cheveux.

4. Composition cosmétique ou dermatologique comprenant au moins un composé de formule générale (I') : 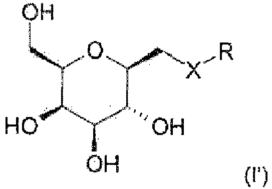 dans lequel :
- X représente un groupe choisi parmi: -CO-,
- CH(NR₁R₂)-, -CHR'- et -C(=CHR')- ;
- R', R₁ et R₂, qui peuvent être identiques ou différents, ont la même définition que celle donnée pour R, et peuvent également représenter un hydrogène et un radical hydroxyle ;
- R représente une chaîne alkyle linéaire ou ramifiée, saturée ou insaturée, ou un cycle cycloalkyle, contenant de 1 à 10 atomes de carbone, ou un radical phényle, ladite chaîne, ledit cycle ou ledit radical pouvant être facultativement substitué par au moins un radical choisi parmi -OR'₁, -NR"'₁R'₂, -COOR"₂ et -CONHR"'₂ ;
- R'₂ et R"'₂, qui peuvent être identiques ou différents, représentent un atome d'hydrogène, ou un radical choisi parmi un radical hydroxyle et un radical alkyle linéaire ou ramifié, saturé ou insaturé contenant de 1 à 8 atomes de carbone ;
- R'₁, R"₁, R"₂ et R"'₁, qui peuvent être identiques ou différents, représentent un atome d'hydrogène, ou un radical choisi parmi un radical alkyle linéaire ou ramifié, saturé ou insaturé contenant de 1 à 8 atomes de carbone avec la limitation suivante :
- R₁ et R₂ ne peuvent pas être simultanément un radical hydroxyle ;
ledit composé étant combiné avec des agents actifs pour les cheveux, choisis parmi :
a) l'acide 13-cis-rétinoïque ou l'acétate de cyprotérone ;
b) le zinc pyrithione, le disulfure de sélénium, le climbazole, l'acide undécylénique, le kétoconazole, la piroctone, l'olamine ou l'eyélopiroctone ;
c) des agents actifs pour stimuler la repousse des cheveux et/ou stimuler le ralentissement de la perte des cheveux choisis parmi :
i) le 3-oxyde de 2,4-diamino-6-pipéridinopyrimidine ou le 3-oxyde de 2,4-diaminopyrimidine ;
ii) la 6-chloro-2,3-dihydroxy-1,4-naphtoquinone, l'oxyde nitrique et des composés donneurs d'oxyde nitrique, le stanozolol, des anthocyanosides, des bioflavonoïdes, FGA ;
d) la cinnarizine, la nimodipine et la nifédipine ;
e) le cromakalim et le nicrorandil.

5. Utilisation cosmétique non thérapeutique d'au moins un composé de formule générale (I') : 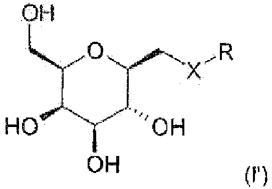 dans lequel :
- X représente un groupe choisi parmi: -CO-,
- CH(NR₁R₂)-, -CHR'- et -C(=CHR')- ;
- R', R₁ et R₂, qui peuvent être identiques ou différents, ont la même définition que celle donnée pour R, et peuvent également représenter un hydrogène et un radical hydroxyle ;
- R représente une chaîne alkyle linéaire ou ramifiée, saturée ou insaturée, ou un cycle cycloalkyle, contenant de 1 à 10 atomes de carbone, ou un radical phényle, ladite chaîne, ledit cycle ou ledit radical pouvant être facultativement substitué par au moins un radical choisi parmi -OR'₁, -NR"'₁R'₂, -COOR"₂ et -CONHR"'₂ ;
- R'₂ et R"'₂, qui peuvent être identiques ou différents, représentent un atome d'hydrogène, ou un radical choisi parmi un radical hydroxyle et un radical alkyle linéaire ou ramifié, saturé ou insaturé contenant de 1 à 8 atomes de carbone ;
- R'₁, R"₁, R"₂ et R"'₁, qui peuvent être identiques ou différents, représentent un atome d'hydrogène, ou un radical choisi parmi un radical alkyle linéaire ou ramifié, saturé ou insaturé contenant de 1 à 8 atomes de carbone
avec la limitation suivante :
- R₁ et R₂ ne peuvent pas être simultanément un radical hydroxyle ;
pour traiter le blanchissement ou le grisonnement prématuré des cheveux.

6. Utilisation selon la revendication 5, **caractérisée en ce que** :
- X représente un groupe choisi parmi : -CO-, -CH(NR₁R₂)- et -CHR' ;
- R représente une chaîne alkyle linéaire ou ramifiée, saturée ou insaturée, ou un cycle cycloalkyle, contenant de 1 à 10 atomes de carbone, ou un radical phényle.

7. Utilisation selon l'une quelconque des revendications 5 à 6, **caractérisée en ce que** ledit composé de formule générale (I') est combiné avec au moins un agent actif pour les cheveux.

8. Utilisation d'au moins un composé de formule générale (I'): 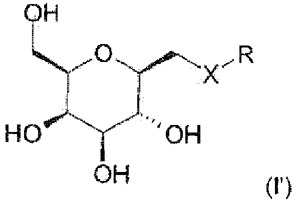 dans lequel :
- X représente un groupe choisi parmi : -CO-, -CH(NR₁R₂)-, -CHR'- et -C(=CHR')-;
- R', R₁ et R₂, qui peuvent être identiques ou différents, ont la même définition que celle donnée pour R, et peuvent également représenter un hydrogène et un radical hydroxyle ;
- R représente une chaîne alkyle linéaire ou ramifiée, saturée ou insaturée, ou un cycle cycloalkyle, contenant de 1 à 10 atomes de carbone, ou un radical phényle, ladite chaîne, ledit cycle ou ledit radical pouvant être facultativement substitué par au moins un radical choisi parmi -OR'₁, -NR'"₁R'₂, -COOR"₂ et -CONHR"'₂ ;
- R'₂ et R"'₂, qui peuvent être identiques ou différents, représentent un atome d'hydrogène, ou un radical choisi parmi un radical hydroxyle et un radical alkyle linéaire ou ramifié, saturé ou insaturé contenant de 1 à 8 atomes de carbone ;
- R'₁, R"₁, R"₂ et R"'₁, qui peuvent être identiques ou différents, représentent un atome d'hydrogène, ou un radical choisi parmi un radical alkyle linéaire ou ramifié, saturé ou insaturé contenant de 1 à 8 atomes de carbone
avec la limitation suivante :
- R₁ et R₂ ne peuvent pas être simultanément un radical hydroxyle ;
pour préparer une composition, comprenant un milieu physiologiquement acceptable, pour utilisation dans la prévention et/ou le traitement de maladies auto-immunes cutanées ou de troubles atopiques cutanés.

9. Utilisation selon la revendication 8, **caractérisée en ce que** :
- X représente un groupe choisi parmi: -CO-, -CH(NR₁R₂)- et -CHR' - ;
- R représente une chaîne alkyle linéaire ou ramifiée, saturée ou insaturée, ou un cycle cycloalkyle, contenant de 1 à 10 atomes de carbone, ou un radical phényle.

10. Utilisation selon l'une quelconque des revendications 8 à 9, **caractérisée en ce que** les troubles atopiques cutanés sont choisis parmi des réactions allergiques cutanées, la dermatite atopique et l'eczéma atopique.

11. Utilisation selon la revendication 10, **caractérisée en ce que** les maladies auto-immunes cutanées sont choisies parmi une hypersensibilité de contact retardée, le psoriasis, le vitiligo, la sclérodermie diffuse, le lupus érythémateux ou certaines formes d'alopécie.

12. Utilisation selon l'une quelconque des revendications 5 à 11, **caractérisée en ce que** ladite composition est destinée à être appliquée de façon topique sur la peau, les membranes muqueuses ou le cuir chevelu.

13. Utilisation d'au moins un composé de formule générale (I') choisi parmi :
Composé 1. 1-(C-β-D-galactopyranosyl)propan-2-one ;
Composé 2. phényl-2-(C-β-D-galactopyranosyl)-1-hydroxyéthane ;
Composé 3. 1-(C-β-D-galactopyranosyl) undécan-2-one ;
Composé 4. 1-[2-(3-hydroxypropylamino)propyl]-C-β-D-galactopyranose ;
Composé 5. ester éthylique d'acide 3-méthyl-4-(C-β-D-galactopyranosyl)-2-buténoïque ;
Composé 6. (2E)-3-méthyl-4-[(2S,3R,4R,5R,6R)-3,4,5-trihydroxy-6-(hydroxyméthyl)tétrahydro-2H-pyran-2-yl]but-2-énoate d'éthyle,
pour traiter le blanchissement ou le grisonnement prématuré des cheveux qui apparaît pendant le vieillissement chronologique ou photo-induit.

14. Utilisation d'au moins un composé de formule générale (I') choisi parmi :
Composé 1. 1-(C-β-D-galactopyranosyl)propan-2-one ;
Composé 2. phényl-2-(C-β-D-galactopyranosyl)-1-hydroxyéthane ;
Composé 3. 1-(C-β-D-galactopyranosyl)undécan-2-one ;
Composé 4. 1-[2-(3-hydroxypropylamino)propyl]-C-β-D-galactopyranose ;
Composé 5. ester éthylique d'acide 3-méthyl-4-(C-β-D-galactopyranosyl)-2-buténoïque ;
Composé 6. (2E)-3-méthyl-4-[(2S,3R,4R,5R,6R)-3,4,5-trihydroxy-6-(hydroxyméthyl)tétrahydro-2H-pyran-2-yl]but-2-énoate d'éthyle,
pour préparer une composition, comprenant un milieu physiologiquement acceptable, pour utilisation dans la prévention et/ou le traitement de maladies auto-immunes cutanées ou de troubles atopiques cutanés.
